# EUROPEAN PATENT SPECIFICATION

(11) **EP 2 266 439 B1**
(45) Date of publication and mention of the grant of the patent: **23.12.2015**
(21) Application number: 09732915.5
(22) Date of filing: 07.04.2009
(51) Int. Cl.: A61M 21/02, A47C 3/025, A61M 21/00

(54) **RELAXATION DEVICE**
ENTSPANNUNGSVORRICHTUNG
DISPOSITIF DE RELAXATION

(30) Priority: 18.04.2008 JP 2008109058
(43) Date of publication of application: 29.12.2010
(73) Proprietor: Panasonic Corporation, Osaka 571-8501 (JP)
(72) Inventor: NISHIO, Fumihiro, Osaka-shi, Osaka 540-6207 (JP); NAKAMURA, Junji, Osaka-shi, Osaka 540-6207 (JP); HAMATSUKA, Taichi, Osaka-shi, Osaka 540-6207 (JP); MORIKAWA, Daisuke, Osaka-shi, Osaka 540-6207 (JP); TANIZAWA, Takayoshi, Osaka-shi, Osaka 540-6207 (JP)
(74) Representative: Appelt, Christian W.
(86) International application number: PCT/JP2009/057091
(87) International publication number: WO 2009/128361

(56) References cited:
- JP-A- 2000 300 374
- JP-A- 2001 149 164
- JP-A- 2001 178 576
- JP-A- 2004 173 865
- US-A- 2 562 324

## Description

### TECHNICAL FIELD

The present invention relates to a relaxation device that has a relaxing effect on a user.

### BACKGROUND ART

Document JP 2000-300374 describes an example of a relaxation device including a rocking driving means, which rocks a body supporting means for supporting a user's body in forward and rearward directions, and a control means, which controls the rocking driving means so as to vary the rocking amplitude of the body supporting means. By changing the rocking amplitude, the relaxation device has a relaxing effect on the user.

In the relaxation device, the rocking driving means includes a motor, which is capable of producing rotation in forward and reverse directions, a speed reducer, which reduces the speed of the drive force generated by the motor, a crank, which is connected to an output shaft of the speed reducer in an integrally rotatable manner, and a rod, which is pivotally connected to a distal end of the crank. The rod has a distal end connected to a base, which is fixed to the body supporting means (seat). The control means produces rotation with the motor alternately in normal and reverse directions within a predetermined range. The speed reducer, crank, and rod transmit the drive force of the motor to the base. This rocks the body supporting means (seat), which is fixed to the base, in forward and rearward directions.

Document US 2 562 324 A discloses a toy apparatus for carrying passengers in which a car is mounted on arms. The arms are driven by a reversible motor, so that they swing to and fro in a gradually increasing arc. The apparatus further comprises brush leads connected to the opposed anchor terminals of a knife switch whereas leads are connected to the switch, so that the follow of current to the brushes is reversed, thereby determining the direction of motor rotation.

### DISCLOSURE OF THE INVENTION

In the above-discussed relaxation device, the control means drives the motor of the rocking driving mean so as to repeatedly produce rotation in normal and reverse directions to rock the body supporting means. Before and after inverting the rotation direction of the motor, the load applied to components, such as the motor, the speed reducer, the rod, and the crank is also inverted in direction. This causes backlash of the components that causes an uncomfortable sensation for the user. In particular, when the rocking is being produced at maximum amplitude, the load is also large. Thus, the discomfort becomes prominent.

It is an object of the present invention to provide a relaxation device that suppresses discomforts and has a further effective relaxing effect on the user. According to the present invention the above object is achieved by a relaxation device according to the characteristics set out in claim 1.

Rotation of the motor is not inverted when the body support means rocks at the maximum amplitude. This prevents the direction of a large load applied to each component of a drive transmission system from being inverted when the rocking amplitude is maximum. Thus, discomfort for the user is reduced when the direction of rocking is inverted, and an effective relaxing effect is provided.

In one aspect, the control means produces alternating normal and reverse rotation with the motor to rock the body supporting means at an amplitude other than the maximum amplitude and decreases rotation speed of the motor as a position at which the rotation direction of the motor is inverted becomes closer. As a result, fluctuations become moderate in the load applied to components during the inversion. This reduces discomfort for the user when the direction of rocking is inverted and provides a further effective relaxing effect.

In one aspect, the control means controls the motor so as to decrease its rotation speed when the rocking driving means is located at a position at which an acceleration load is received from the body supporting means, and the control means controls the motor so as to increase its rotation speed when the rocking driving means is located at a position at which a deceleration load is received from the body supporting means.

When the motor produces alternating normal and reverse rotation and when the motor produces single-direction rotation, which is the state in which the amplitude is maximum, the rocking speed has a tendency to become unstable due to the level of the load on the body support. However, the rotation speed of the motor is adjusted in accordance with the load level of the body support to stabilize the rocking speed. Thus, a further effective relaxing effect is provided.

In a further aspect, the control means temporarily stops the motor at a position at which rotation is inverted when producing alternating normal and reverse rotation with the motor to rock the body supporting means at an amplitude other than the maximum amplitude, and the control means starts to drive and invert rotation of the motor after the body supporting means start to invert rotation due to its own weight. This drives and inverts rotation of the motor in accordance with the rocking movement resulting from the weight of the body supporting means. Thus, the rocking inversion movement is smooth. This provides a further effective relaxing effect.

### BRIEF DESCRIPTION OF THE DRAWINGS

Fig. 1 is a schematic diagram showing a relaxation device according to one embodiment;
Figs. 2(a) to 2(d) are diagrams showing operational zones of a crank;
Fig. 3 is a chart showing the movement speed of the crank;
Figs. 4(a) to 4(c) are diagrams showing rotation of the crank and rocking of the base; and
Figs. 5(a) to 5(d) are diagrams showing the movement of the crank in a further example.

### BEST MODE FOR CARRYING OUT THE INVENTION

One embodiment of the present invention will now be discussed with reference to the drawings.

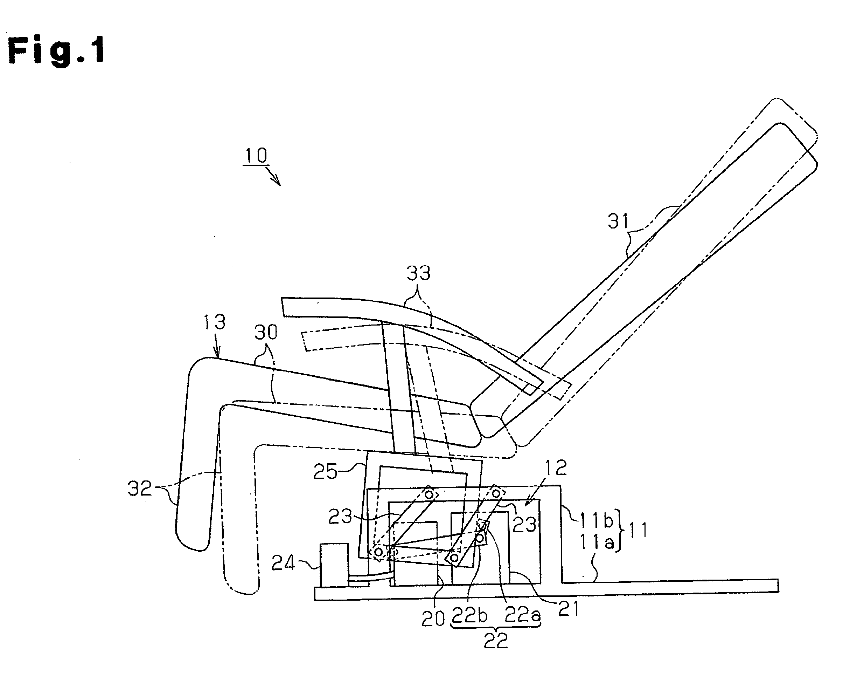

Fig. 1 is a schematic diagram showing a relaxation device 10 of the present embodiment. As shown in Fig. 1, the relaxation device 10 includes a rack 11 having a bottom portion 11a, which is arranged on a floor surface (not shown). A rocking driver 12, which serves as a rocking driving means, is arranged in the rack 11. The rocking driver 12 rocks a body support 13, which serves as a body supporting means.

The rocking driver 12 includes a motor 20, a speed reducer 21, a crank mechanism 22, and links 23.

The motor 20 is arranged on an upper surface of the bottom portion 11a of the rack 11. A controller 24, which serves as a control means (also referred to as a main control means) and a refreshment control means, is arranged on the upper surface of the bottom portion 11a. The controller 24 controls and drives the motor 20. The speed reducer 21 is arranged on the upper surface of the bottom portion 11a of the rack 11. The speed reducer 21 is connected to the motor 20 and outputs the drive force from the motor 20 at a reduced speed.

The crank mechanism 22, which includes a crank 22a and a rod 22b, converts the rotational drive of the speed reducer 21 to an arced motion of which the diameter is extremely large. The crank 22a has a basal end connected to an output shaft 21a of the speed reducer 21 in an integrally rotatable manner. The crank 22a has a distal end connected in a rotatable manner to a basal end of the rod 22b. The rod 22b has a distal end connected to a lower portion of a tetragonal frame-shaped base 25 fixed to the body support 13.

The rack 11 includes a support frame 11b, extending upward from the bottom portion 11a. The two links 23 have basal ends connected in a rotatable manner to an upper portion of the support frame 11b and spaced apart from each other by a predetermined distance. Further, the two links 23 have distal ends connected in a rotatable manner to a lower portion of the base 25. Thus, the two links 23 are each pivoted (swung) about its basal end and rock the body support 13, which is fixed to the base 25, in forward and rearward directions like a rocking chair when receiving drive force from the crank mechanism 22. The crank 22a, the rod 22b, and the links 23 serve as a rocking driving means in addition to serving as a conversion mechanism.

The chair-shaped body support 13 is fixed to the upper portion of the base 25. The body support 13 includes a seat 30, a backrest 31, a footrest 32, and armrests 33. The seat 30 is fixed to the base 25 so as to move integrally with the base 25. The inclinable backrest 31 is fixed to a rear part of the seat 30. The footrest 32 is fixed to a front part of the seat 30. The armrests 33 are arranged at the left and right sides of the seat 30.

A process for controlling the relaxation device 10 will now be discussed with reference to Figs. 1 to 4.

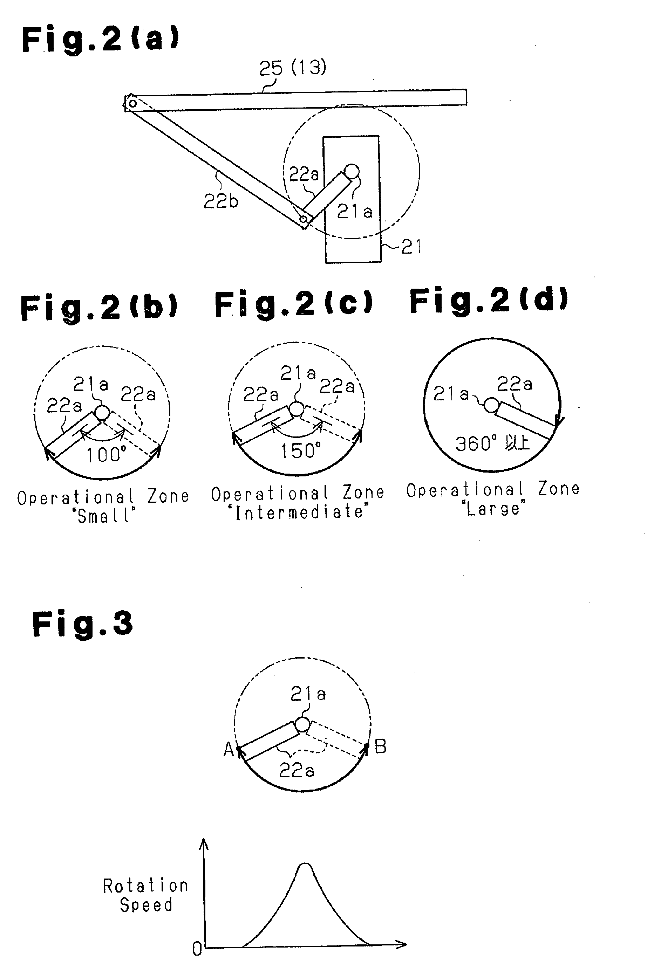

The controller 24 controls the motor 20 to rock the body support 13 with rocking widths (amplitude) in a plurality of stages (three stages in the present embodiment). In the example shown in Fig. 1 and Figs. 2(a) to 2(d), the rocking amplitude of the body support 13 is set in three stages. More specifically, the controller 24 controls the motor 20 and pivots the crank 22a, which is connected to the output shaft 21a of the speed reducer 21, so that the crank 22a is operated in three operational zones, which are defined as "small," "intermediate," and "large." In correspondence with the operational zones of the crank 22a, the body support 13 is rocked at amplitudes set in three stages. The controller 24 controls the motor 20 to vary the operational zone for the rocking of the body support 13. In the description of the present embodiment, the operational zones of the crank 22a conform to the operational zones of the motor 20.

Referring to Figs. 2(b) and 2(c), to rock the body support 13, the controller 24 produces alternating normal rotation and reverse rotation with the motor 20, which is capable of producing rotation in normal and reverse directions. That is, the controller 24 inverts the rotation of the motor 20 in a predetermined range so that, for example, the rotational angular range for the output shaft 21a of the speed reducer 21 is approximately 100° when the operational zone of the crank 22a is "small," and the rotational angular range for the output shaft 21a of the speed reducer 21 is approximately 150° when the operational zone is "intermediate." Further, referring to Fig. 2(d), to move the body support 13 so that the amplitude of the rocking is maximum, the controller 24 produces rotation with the motor 20 in a single direction without inverting it when the operational zone of the crank 22a is "maximum," that is, when the rotational angle of the output shaft 21a in the speed reducer 21 is 360° or greater. In the illustrated example, the motor 20 produces rotation in only the normal direction when the operational zone is "large."

In situations in which the rotation of the motor 20 is inverted such as when the operational zone of the crank 22a is "small" or "intermediate," the controller 24 controls the motor 20 to decrease the rotation speed as any one of the two inverting positions of the motor 20 becomes closer (for the present embodiment, in the example shown in Fig. 3, starting point A and ending point B in the operational zone of the crank 22a).

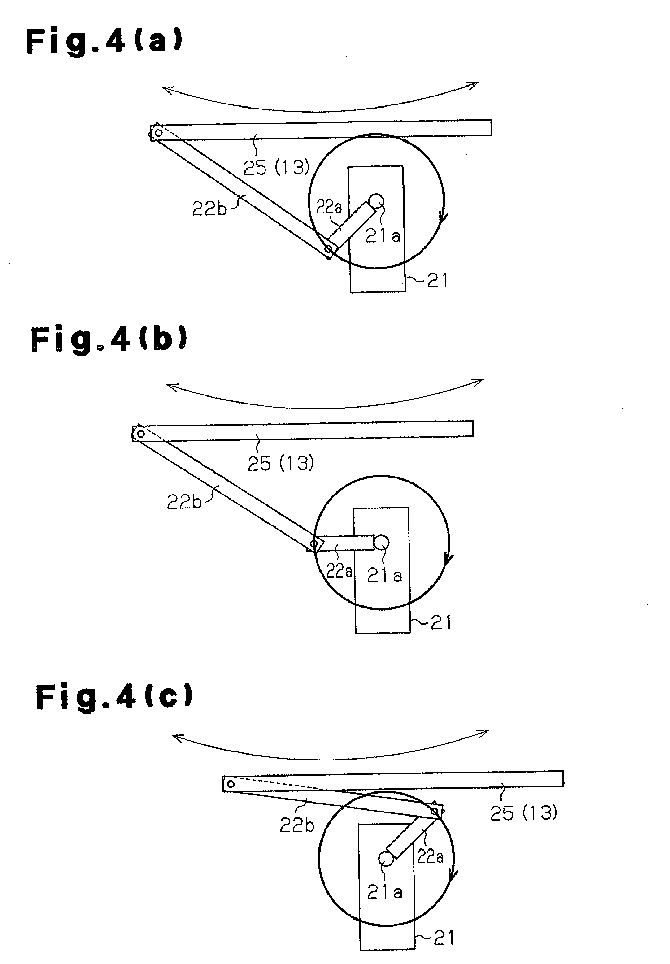

Referring to Figs. 4(a) to 4(c), the controller 24 controls the rotation speed of the motor 20 (see Fig. 1) in accordance with the load applied to the base 25, which is fixed to the body support 13 (see Fig. 1) to appropriately pivot the crank 22a and rock the body support 13. In the illustrated example, when in the state of Fig. 4(c), the body support 13 applies load, which would accelerate rotation, to the motor 20 and the crank 22a. In this case, the controller 24 controls the rotation produced by the motor 20 so as to decrease the rotation speed. In the state shown in Figs. 4(a) and 4(b), the body support 13 applies load to the motor 20 and the crank 22a and hinders rotation. In this case, the controller 24 controls the motor 20 to increase the rotation speed. This stably rocks the body support 13 regardless of the load from the body support 13.

The speed control is performed in accordance with detection signals of the speed or position of the crank 22a and output shaft 21a. In a further example, the speed control is performed in accordance with detection signals of the speed or position of the body support 13.

Fig. 4(c) shows a state in which the rocking driving means is located at a position at which it receives an acceleration load from the body supporting means. Figs. 4(a) and 4(b) each show a state in which the rocking driving means is located at a position at which it receives a deceleration load from the body supporting means.

In the relaxation device 10 of the present embodiment, the controller 24 executes combinations of the speed controls described above on the motor 20. This rocks the body support 13 in forward and rearward directions to perform relaxation rocking that has an effective relaxing effect on the user.

The advantages of the present embodiment will now be described.
(1) The rocking driver 12 includes the crank 22a, the rod 22b, and the links 23, which convert both alternating normal and reverse rotation and single-direction rotation produced by the motor 20 to the rocking movement of the body support 13. When the rocking amplitude of the body support 13 is maximum (the operational zone of the crank 22a is "large"), the controller 24 produces rotation in only one direction with the motor 20. The rotation of the motor 20 is not inverted when the rocking amplitude of the body support 13 is maximum. This prevents the direction of a large load applied to each component of the drive transmission system, such as the crank 22a and the rod 22b, from being inverted when the rocking amplitude is maximum. Thus, discomfort of the user is reduced when the direction of rocking is inverted, and an effective relaxing effect is provided.
(2) The controller 24 alternately produces normal rotation and reverse rotation with the motor 20 when rocking is performed at an amplitude other than the maximum amplitude (operational zones of the crank member being "small" or "intermediate"). However, the controller 24 controls the rotation speed of the motor 20 so that the rotation speed of the motor 20 decreases as an inverting position in the operational zone of the motor 20 becomes closer. By decreasing the rotation speed of the motor 20 immediately before and after the inversion, fluctuations become moderate in the load applied to components such as the crank 22a and the rod 22b during the inversion. This reduces discomfort for the user when the direction of rocking is inverted and provides a further effective relaxing effect.
(3) The controller 24 controls and decreases the rotation speed of the motor 20 when the rocking driving means is located at a position at which it receives an acceleration load from the body support 13. Further, the controller 24 controls and increases the rotation speed of the motor 20 when the rocking driving means is located at a position at which it receives a deceleration load from the body support 13. When the motor 20 produces alternating normal and reverse rotation and when the motor 20 produces single-direction rotation, which is the state in which the amplitude is maximum, the rocking speed has a tendency to become unstable due to the load on the body support 13. However, the rotation speed of the motor 20 is adjusted in accordance with the level of the load on the body support 13 to stabilize the rocking speed. Thus, a further effective relaxing effect is provided.

The embodiment of the present invention may be modified as described below.

In the above-discussed embodiment, the rotation speed of the motor 20 is varied in accordance with the distance from the inverting position at which the rotation direction of the crank 22a is changed (inversion position of the motor 20). However, the present invention is not limited in such a manner. For example, the rotation speed of the motor may be kept constant and not be varied.

In the above-discussed embodiment, the rotation speed of the motor 20 is varied in accordance with the direction of the load applied on the rocked body support but does not have to be varied.

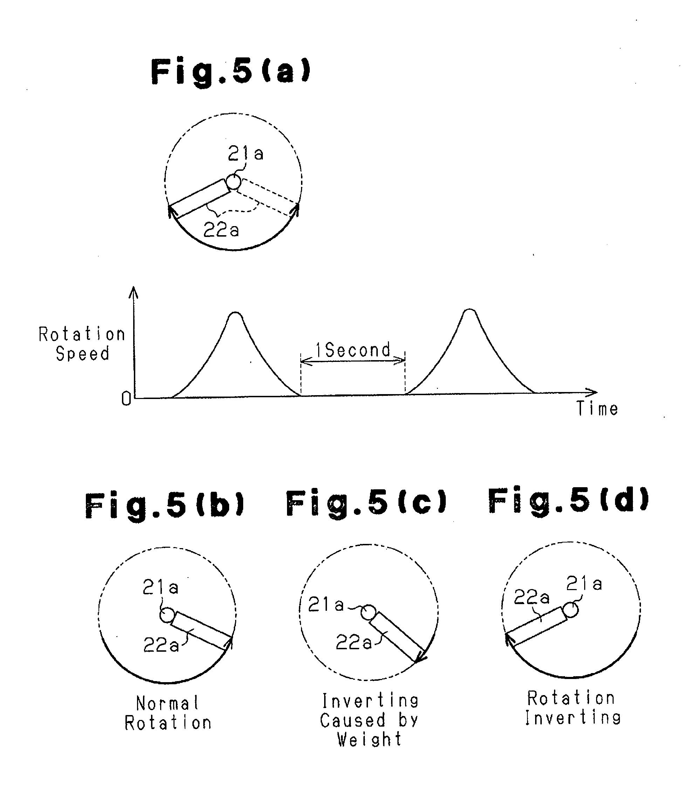

Although not particularly mentioned, as shown in Figs. 5(a) to 5(d), during alternating normal and reverse rotation of the motor, the controller 24 may temporarily (e.g., one second) stop the motor 20 when inverting rotation of the crank member 22a. After the body support 13 starts the inversion with its own weight during the stoppage period, the controller 24 may control the motor 20 again so that it is driven in an inverted direction. This inverts the driving of the motor in accordance with the rocking caused by the weight of the crank 22a. This provides a further effective relaxing effect.

In the above-discussed embodiment, the body support 13 is chair-shaped. However, the present invention is not limited in such a manner. For example, the body support 13 may be bed-shaped or have any other shape.

In the above-discussed embodiment, the body support 13 is rocked in forward and rearward directions but is not limited in such a manner and may be rocked in sideward directions.

## Claims

1. A relaxation device (10) including:
a body supporting means (13) for supporting a user's body;
a rocking driving means (12), including a motor (20) capable of producing normal rotation and reverse rotation, for rocking the body supporting means (13) by driving and producing rotation with the motor (20); and
a control means (24) for controlling rotation of the motor (20) to rock the body supporting means (13);
the relaxation device (10) being **characterized in that**: the rocking driving means (12) includes
a speed reducer (21) connected to the motor (20) for outputting the drive force from the motor 20 at a reduced speed, and
a conversion mechanism that converts both alternating normal and reverse rotation and single-direction rotation into a rocking movement of the body supporting means (13);
wherein the conversion mechanism includes
a crank mechanism (22) including a crank (22a), a rod (22b) and links (23),
wherein the crank (22a) has a basal end connected to an output shaft (21a) of the speed reducer (21) in an integrally rotatable manner, and the crank (22a) has a distal end connected in a rotatable manner to a basal end of the rod (22b),
the control means (24) controls the motor (20) to produce rotation in a single direction when rocking the body supporting means (13) at maximum amplitude.

2. The relaxation device (10) according to claim 1, being **characterized in that**:
the control means (24) produces alternating normal and reverse rotation with the motor (20) to rock the body supporting means (13) at an amplitude other than the maximum amplitude and decreases rotation speed of the motor (20) as a position at which the rotation direction of the motor (20) is inverted becomes closer.

3. The relaxation device (10) according to claim 1, being **characterized in that**:
the control means (24) controls the motor (20) so as to decrease its rotation speed when the rocking driving means (12) is located at a position at which an acceleration load is received from the body supporting means (13), and the control means (24) controls the motor (20) so as to increase its rotation speed when the rocking driving means (12) is located at a position at which a deceleration load is received from the body supporting means (13).

4. The relaxation device (10) according to any one of claims 1 to 3, being **characterized in that**:
the control means (24) temporarily stops the motor (20) at a position at which rotation is inverted when producing alternating normal and reverse rotation with the motor (20) to rock the body supporting means (13) at an amplitude other than the maximum amplitude, and the control means (24) starts to drive and invert rotation of the motor after the body supporting means (13) start to invert rotation due to its own weight.

## Patentansprüche

1. Entspannungsvorrichtung (10), umfassend:
ein Körperstützmittel (13) zum Tragen des Körpers eines Benutzers;
ein Schaukelantriebsmittel (12) mit einem Motor (20), welcher eine normale Drehung und eine entgegengesetzte Drehung erzeugen kann, zum Schaukeln des Körperstützmittels (13) durch Antreiben und Erzeugen einer Drehung mit dem Motor (20); und
ein Steuermittel (24) zum Steuern der Drehung des Motors (20), um das Körperstützmittel (13) zu schaukeln;
wobei die Entspannungsvorrichtung (10) **dadurch gekennzeichnet ist, dass**: das Schaukelantriebsmittel (12) umfasst
ein Untersetzungsgetriebe (21), welches mit dem Motor (20) verbunden ist, um die Antriebskraft vom Motor (20) mit verringerter Drehzahl auszugeben, und
einen Umwandlungsmechanismus, welcher sowohl eine abwechselnde normale und entgegengesetzte Drehung als auch eine Drehung in eine einzige Richtung in eine Schaukelbewegung des Körperstützmittels (13) umwandelt;
wobei der Umwandlungsmechanismus umfasst
einen Kurbelwellenmechanismus (22) mit einer Kurbel (22a), mit einer Stange (22b) und mit Gestängen (23),
wobei die Kurbel (22a) ein Grundende aufweist, das mit einer Abtriebswelle (21a) des Untersetzungsgetriebes (21) in einer integral drehbaren Weise verbunden ist, und die Kurbel (22a) ein distales Ende aufweist, das in einer drehbaren Weise mit einem Grundende der Stange (22b) verbunden ist,
wobei das Steuermittel (24) den Motor (20) steuert, um eine Drehung in eine einzige Richtung zu erzeugen, wenn das Körperstützmittel (13) mit maximaler Amplitude geschaukelt wird.

2. Entspannungsvorrichtung (10) nach Anspruch 1, **dadurch gekennzeichnet, dass**:
das Steuermittel (24) abwechselnde normale und entgegengesetzte Drehung mit dem Motor (20) erzeugt, um das Körperstützmittel (13) mit einer anderen Amplitude als die maximale Amplitude zu schaukeln, und die Drehzahl des Motors (20) verringert, wenn eine Position näherkommt, bei welcher die Drehrichtung des Motors (20) umgekehrt wird.

3. Entspannungsvorrichtung (10) nach Anspruch 1, **dadurch gekennzeichnet, dass**:
das Steuermittel (24) den Motor (20) steuert, um seine Drehzahl zu verringern, wenn das Schaukelantriebsmittel (12) in einer Position angeordnet ist, in welcher eine Beschleunigungslast vom Körperstützmittel (13) empfangen wird, und das Steuermittel (24) den Motor (20) steuert, um so seine Drehzahl zu erhöhen, wenn das Schaukelantriebsmittel (12) in einer Position angeordnet ist, in welcher eine Verzögerungslast vom Körperstützmittel (13) empfangen wird.

4. Entspannungsvorrichtung (10) nach einem beliebigen der Ansprüche 1 bis 3, **dadurch gekennzeichnet, dass**:
das Steuermittel (24) den Motor (20) in einer Position vorübergehend anhält, in welcher die Drehung umgekehrt wird, wenn mit dem Motor (20) abwechselnd normale und entgegengesetzte Drehung erzeugt wird, um das Körperstützmittel (13) mit einer Amplitude, welche nicht die maximale Amplitude ist, zu schaukeln, und das Steuermittel (24) beginnt, die Drehung des Motors anzutreiben und umzukehren, nachdem das Körperstützmittel (13) beginnt, aufgrund des eigenen Gewichts die Drehung umzukehren.

## Revendications

1. Dispositif de relaxation (10) comprenant :
des moyens de support de corps (13) pour supporter le corps d'un utilisateur ;
des moyens d'entraînement de basculement (12), comprenant un moteur (20) capable de produire une rotation normale et une rotation inverse, pour basculer les moyens de support de corps (13) par l'entraînement et la production d'une rotation par le moteur (20) ; et
des moyens de commande (24) pour commander la rotation du moteur (20) pour basculer les moyens de support de corps (13) ;
le dispositif de relaxation (10) étant **caractérisé en ce que** : les moyens d'entraînement de basculement (12) comprennent :
un réducteur de vitesse (21) relié au moteur (20) pour délivrer la force d'entraînement du moteur (20) à une vitesse réduite, et
un mécanisme de conversion qui convertit à la fois les rotations normale et inverse alternées et une rotation dans une direction unique en un mouvement de basculement des moyens de support de corps (13) ;
dans lequel le mécanisme de conversion comprend :
un mécanisme à vilebrequin (22) comprenant un vilebrequin (22a), une bielle (22b) et des liaisons (23),
dans lequel le vilebrequin (22a) a une extrémité de base reliée à un arbre de sortie (21a) du réducteur de vitesse (21) en rotation intégrale, et le vilebrequin (22a) a une extrémité distale reliée en rotation à une extrémité de base de la bielle (22b),
les moyens de commande (24) commandent le moteur (20) pour produire une rotation dans une direction unique lors du basculement des moyens de support de corps (13) à une amplitude maximale.

2. Dispositif de relaxation (10) selon la revendication 1, **caractérisé en ce que** :
les moyens de commande (24) produisent des rotations normale et inverse par le moteur (20) pour basculer les moyens de support de corps (13) à une amplitude autre que l'amplitude maximale et diminuent la vitesse de rotation du moteur (20) alors qu'une position à laquelle la direction de rotation du moteur (20) est inversée devient plus proche.

3. Dispositif de relaxation (10) selon la revendication 1, **caractérisé en ce que** :
les moyens de commande (24) commandent le moteur (20) de manière à diminuer sa vitesse de rotation lorsque les moyens d'entraînement de basculement (12) sont situés à une position à laquelle une charge d'accélération est reçue des moyens de support de corps (13), et les moyens de commande (24) commandent le moteur (20) de manière à augmenter sa vitesse de rotation lorsque les moyens d'entraînement de basculement (12) sont situés à une position à laquelle une charge de décélération est reçue des moyens de support de corps (13).

4. Dispositif de relaxation (10) selon l'une quelconque des revendications 1 à 3, **caractérisé en ce que** :
les moyens de commande (24) arrêtent temporairement le moteur (20) à une position à laquelle la rotation est inversée lors de la production de rotations normale et inverse alternées par le moteur (20) pour basculer les moyens de support de corps (13) à une amplitude autre que l'amplitude maximale, et les moyens de commande (24) débutent l'entraînement et l'inversion de la rotation du moteur après que les moyens de support de corps (13) ont débuté l'inversion de la rotation du fait de leur propre poids.
